# EUROPEAN PATENT SPECIFICATION

(11) **EP 2 478 907 B1**
(45) Date of publication and mention of the grant of the patent: **14.04.2021**
(21) Application number: 12160586.9
(22) Date of filing: 23.08.2007
(51) Int. Cl.: A61K 31/58, A61K 31/573, A61P 35/00, A61K 45/06, A61P 13/08, A61P 43/00

(54) **Compositions for treating cancer**
Zusammensetzungen zur Behandlung von Krebs
Compositions pour le traitement du cancer

(30) Priority: 25.08.2006 US 921506 P
(43) Date of publication of application: 25.07.2012
(62) Divisional of application: 07837326.3
(73) Proprietor: Janssen Oncology, Inc., Los Angeles CA 90024 (US)
(72) Inventor: Auerbach, Alan H., Los Angeles, CA 90024 (US); Belldegrun, Arie S., Los Angeles, CA 90024 (US)
(74) Representative: Carpmaels & Ransford LLP

(56) References cited:
- WO-A-02/053138
- WO-A-2004/037269
- WO-A-2006/021776
- US-A1- 2006 030 608
- O'DONNELL A ET AL: "Hormonal impact of the 17[alpha]-hydroxylase/C17,20-lyase inhibitor abiraterone acetate (CB7630) in patients with prostate cancer", BRITISH JOURNAL OF CANCER 14 JUN 2004 UNITED KINGDOM, vol. 90, no. 12, 14 June 2004 (2004-06-14) , pages 2317-2325, XP002464868, ISSN: 0007-0920
- MADAN RAVI A ET AL: "Abiraterone Cougar Biotechnology", IDRUGS, CURRENT DRUGS LTD, GB, vol. 9, no. 1, January 2006 (2006-01), pages 49-55, XP008087480, ISSN: 1369-7056
- SCHOLZ M ET AL: "LONG-TERM OUTCOME FOR MEN WITH ANDROGEN INDEPENDENT PROSTATE CANCER TREATED WITH KETOCONAZOLE AND HYDROCORTISONE", JOURNAL OF UROLOGY, LIPPINCOTT WILLIAMS & WILKINS, BALTIMORE, MD, US, vol. 173, no. 6, 1 June 2005 (2005-06-01), pages 1947-1952, XP027855453, ISSN: 0022-5347 [retrieved on 2005-06-01]
- HARRIS ET AL: "Low Dose Ketoconazole With Replacement Doses of Hydrocortisone in Patients With Progressive Androgen Independent Prostate Cancer", JOURNAL OF UROLOGY, LIPPINCOTT WILLIAMS & WILKINS, BALTIMORE, MD, US, vol. 168, no. 2, 1 August 2002 (2002-08-01), pages 542-545, XP005540121, ISSN: 0022-5347, DOI: 10.1016/S0022-5347(05)64675-7
- GERBER G S ET AL: "Prostate specific antigen for assessing response to ketoconazole and prednisone in patients with hormone refractory metastatic prostate cancer", JOURNAL OF UROLOGY, LIPPINCOTT WILLIAMS & WILKINS, BALTIMORE, MD, US, vol. 144, no. 5, 1 January 1990 (1990-01-01), pages 1177-1179, XP008151701, ISSN: 0022-5347
- DE COSTER R ET AL: "EFFECTS OF HIGH-DOSE KETOCONAZOLE AND DEXAMETHASONE ON ACTH-STIMULATED ADRENAL STEROIDOGENESIS IN ORCHIECTOMIZED PROSTATIC CANCER PATIENTS", ACTA ENDOCRINOLOGICA, vol. 115, no. 2, 1987, pages 265-271, XP008153090, ISSN: 0001-5598
- FOSSA ET AL: "Weekly Docetaxel and Prednisolone versus Prednisolone Alone in Androgen-Independent Prostate Cancer: A Randomized Phase II Study", EUROPEAN UROLOGY, ELSEVIER BV, NL, vol. 52, no. 6, 21 November 2007 (2007-11-21), pages 1691-1699, XP022356374, ISSN: 0302-2838, DOI: 10.1016/J.EURURO.2007.01.104
- DANILA DANIEL C ET AL: "Phase II multicenter study of abiraterone acetate plus prednisone therapy in patients with docetaxel-treated castration-resistant prostate cancer.", JOURNAL OF CLINICAL ONCOLOGY : OFFICIAL JOURNAL OF THE AMERICAN SOCIETY OF CLINICAL ONCOLOGY 20 MAR 2010, vol. 28, no. 9, 20 March 2010 (2010-03-20) , pages 1496-1501, ISSN: 1527-7755
- Anonymous: "NCT00485303 on 2007_06_13: ClinicalTrials.gov Archive", , 13 June 2007 (2007-06-13), pages 1-3, XP055394875, Retrieved from the Internet: URL:https://clinicaltrials.gov/archive/NCT 00485303/2007_06_13 [retrieved on 2017-07-31]
- LAM J S ET AL: "Secondary Hormonal Therapy for Advanced Prostate Cancer", JOURNAL OF UROLOGY, LIPPINCOTT WILLIAMS & WILKINS, BALTIMORE, MD, US, vol. 175, no. 1, 1 January 2006 (2006-01-01), pages 27-34, XP024990140, ISSN: 0022-5347, DOI: 10.1016/S0022-5347(05)00034-0 [retrieved on 2006-01-01]
- Garnick M B, Motta C: "Androgen deprivation therapy:the future 93" In: Kirby R S, Partin A W, Feneley M, Parsons J K: "Prostatce Cancer - Principles and Practice", 13 January 2006 (2006-01-13), CRC Press Taylor & Francis Group ISBN: 9781841844589 pages 915-928,
- FAKIH MARWAN ET AL: "Glucocorticoids and treatment of prostate cancer: A preclinical and clinical review", UROLOGY, BELLE MEAD, NJ, US, vol. 60, no. 4, 1 October 2002 (2002-10-01), pages 553-561, XP002495992, ISSN: 0090-4295, DOI: 10.1016/S0090-4295(02)01741-7
- HERR I ET AL: "Glucocorticoid use in prostate cancer and other solid tumours: implications for effectiveness of cytotoxic treatment and metastases", THE LANCET ONCOLOGY, ELSEVIER, AMSTERDAM, NL, vol. 7, no. 5, 1 May 2006 (2006-05-01), pages 425-430, XP028057866, ISSN: 1470-2045, DOI: 10.1016/S1470-2045(06)70694-5 [retrieved on 2006-05-01]
- RUTZ H P: "Effects of corticosteroid use on treatment of solid tumours", THE LA, THE LANCET PUBLISHING GROUP, GB, vol. 360, no. 9349, 14 December 2002 (2002-12-14), pages 1969-1970, XP004795228, ISSN: 0140-6736, DOI: 10.1016/S0140-6736(02)11922-2
- ATTARD G ET AL: "Selective blockade of androgenic steroid synthesis by novel lyase inhibitors as a therapeutic strategy for treating metastatic prostate cancer", BJU INTERNATIONAL, BLACKWELL SCIENCE, OXFORD, GB, vol. 96, 1 January 2005 (2005-01-01), pages 1241-1246, XP002497670, ISSN: 1464-4096, DOI: 10.1111/J.1464-410X.2005.05821.X
- Anonymous: "Summary of product characteristics for Zytiga 250 mg tablets" In: "Summary of product characteristics for Zytiga 250 mg tablets", 21 January 2014 (2014-01-21), XP055750423,
- GOLDSMITH O ET AL: "Hypogonadism and mineralocorticoid excess. the 17-hydroxylase deficiency syndrome", NEW ENGLAND JOURNAL OF MEDICINE (PRINT) 1967, vol. 277, no. 13, 1967, page 614, ISSN: 0028-4793

## Description

### FIELD OF THE INVENTION

Compositions for treating cancer are described herein. More particularly, the compositions for treating cancer comprise a 17α-hydroxylase/C₁₇₋₂₀-lyase inhibitor, such as abiraterone acetate (i.e., 3β-acetoxy-17-(3-pyridyl) androsta-5,16-diene), in combination with prednisone.

### BACKGROUND

The number of people diagnosed with cancer has significantly increased. Of special interest are individuals diagnosed with androgen-dependent disorders, such as prostate cancer, and estrogen-dependent disorders, such as breast cancer since such diagnoses are increasing in number at an alarming rate.

Prostate cancer is currently the most common non-skin cancer and the second leading cause of cancer-related death in men after lung cancer. The primary course of treatment for patients diagnosed with organ-confined prostate cancer is usually prostatectomy or radiotherapy. Not only are these treatments highly invasive and have undesirable side effects, such localized treatments are not effective on prostate cancer after it has metastasized. Moreover, a large percent of individuals who receive localized treatments will suffer from recurring cancer.

Additionally, breast cancer incidence in women has increased from one out of every 20 women in 1960 to one out of every eight women in 2005. Moreover, it is the most common cancer among white and African-American women. Similar to treating prostate cancer, most options for women diagnosed with breast cancer are highly invasive and have significant side-effects. Such treatments include surgery, radiation and chemotherapy.

Hormone therapy is another treatment option for individuals diagnosed with prostate or breast cancer. Hormone therapy is a form of systemic treatment for prostate or breast cancer wherein hormone ablation agents are used to suppress the production or block the effects of hormones, such as estrogen and progesterone in the body, which are believed to promote the growth of breast cancer, as well as testosterone and dihydrotestosterone, which are believed to promote the growth of prostate cancer. Moreover, hormone therapy is less invasive than surgery and does not have many of the side effects associated with chemotherapy or radiation. Hormone therapy can also be used by itself or in addition to localized therapy and has shown to be effective in individuals whose cancer has metastasized.

Even though hormone therapy is less invasive and can be used on more advanced stages of cancer, some individuals administered current hormone therapy treatments may not show a significant response or may not show any response at all to such treatments. Additionally, some patients treated with current hormone therapy treatments may also suffer from relapsing or recurring cancer. Currently, such refractory cancer patients are left with very few treatment options O'Donnell et al (British Journal of Cancer, 2004, 90, 2317-2325) discusses the hormonal impact of abiraterone acetate in patients with prostate cancer.

Despite the progress made in the treatment of cancer, there remains a need for more effective ways to treat cancer such as, but not limited to, prostate cancer and breast cancer. Additionally, there is a need for effective anti-cancer treatment options for patients who are not responding to current anti-cancer treatments. Also, there is a need for effective anti-cancer treatment options for patients whose cancer has recurred.

### SUMMARY OF THE INVENTION

The invention is defined by the claims. Any subject-matter falling outside the scope of the claims is provided for information purposes only.

The invention provides abiraterone acetate for use in a method of treating prostate cancer in a human, said method comprising administering to said human a therapeutically effective amount of abiraterone acetate and a therapeutically effective amount of prednisone.

The invention also provides prednisone for use in a method of treating prostate cancer in a human, said method comprising administering to said human a therapeutically effective amount of said prednisone and a therapeutically effective amount of abiraterone acetate.

The invention also provides a pharmaceutical composition for use in a method of treating prostate cancer in a human, wherein the pharmaceutical composition comprises a therapeutically effective amount of abiraterone acetate and a therapeutically effective amount of prednisone.

Described herein are methods for treating a cancer in which a therapeutically effective amount of a 17α-hydroxylase/C₁₇₋₂₀-lyase inhibitor, such as abiraterone acetate (i.e. 3β-acetoxy-17-(3-pyridyl) androsta-5,16-diene), is administered to a patient, e,g., a patient in need thereof, in combination with a therapeutically effective amount of at least one additional therapeutic agent such as a steroid. Such methods can also provide an effective treatment for individuals with a refractory cancer, including individuals who are currently undergoing a cancer treatment. Therefore, the methods might be directed to treating a refractory cancer in a patient, in which a therapeutically effective amount of 17α-hydroxylase/C₁₇₋₂₀-lyase inhibitor is administered to a patient currently receiving an anti-cancer agent.

The composition for the treatment of a cancer in a mammal can comprise administering an amount of about 50 mg/day to about 2000 mg/day of abiraterone acetate and an amount of about 0.01 mg/day to about 500 mg/day of a glucocorticoid such as prednisone.

Also described herein are compositions for the treatment of cancer that comprise a combination of a therapeutically effective amount of at least one 17α-hydroxylase/C₁₇₋₂₀-lyase inhibitor and a therapeutically effective amount of at least one steroid such as prednisone.

Finally, single unit dosage forms comprising abiraterone acetate and a glucocorticoid, optionally with carriers, diluents or excipients, are contemplated. Also, kits comprising at least one 17α-hydroxylase/C₁₇₋₂₀-lyase inhibitor and a steroid are contemplated. For example, the kit may include a vial containing abiraterone acetate and another vial containing a glucocorticoid.

### Definitions

As used herein and unless otherwise defined the word "cancer," refers to the growth, division or proliferation of abnormal cells in the body. Cancers that can be treated with the methods and the compositions described herein include, but are not limited to, prostate cancer, breast cancer, adrenal cancer, leukemia, lymphoma, myeloma, Waldenstrom's macroglobulinemia, monoclonal gammopathy, benign monoclonal gammopathy, heavy chain disease, bone and connective tissue sarcoma, brain tumors, thyroid cancer, pancreatic cancer, pituitary cancer, eye cancer, vaginal cancer, vulvar cancer, cervical cancer, uterine cancer, ovarian cancer, esophageal cancer, stomach cancer, colon cancer, rectal cancer, liver cancer, gallbladder cancer, cholangiocarcinoma, lung cancer, testicular cancer, penal cancer, oral cancer, skin cancer, kidney cancers, Wilms' tumor and bladder cancer.

As used herein, and unless otherwise defined, the terms "treat," "treating" and "treatment" include the eradication, removal, modification, management or control of a tumor or primary, regional, or metastatic cancer cells or tissue and the minimization or delay of the spread of cancer.

As used herein, and unless otherwise defined, the term "patient" means an animal, including but not limited to an animal such as a human, monkey, cow, horse, sheep, pig, chicken, turkey, quail, cat, dog, mouse, rat, rabbit, or guinea pig. In one aspect of the disclosure, the patient is a mammal. In the present invention, the patient is a human. In certain embodiments, the patient can be an adult male. In some embodiments, the patient is a male of age about 30 years to about 85 years. In a particular embodiment, the patient has or is susceptible to having (*e.g*., through genetic or environmental factors) cancer. In a further embodiment, the patient has or is susceptible to having (*e.g*., through genetic or environmental factors) a tumor. In other embodiments, the patient can be castrated or non-castrated.

The term " 17α-hydroxylase/C₁₇₋₂₀-lyase inhibitor" as used herein refers to an inhibitor of 17α-hydroxylase/C₁₇₋₂₀-lyase inhibitor, (which is an enzyme in testosterone synthesis) or pharmaceutically acceptable salt thereof.

The term "anti-cancer agent" as used herein refers to any therapeutic agent that directly or indirectly kills cancer cells or directly or indirectly prohibits stops or reduces the proliferation of cancer cells. It should be noted that even though throughout this specification and in the claims the phrase "anti-cancer agent" is written as a singular noun, for example; "an anti-cancer agent" or "the anti-cancer agent," the phrase "anti-cancer agent" should not be interpreted as being limited to the inclusion of a single anti-cancer agent.

As used herein, and unless otherwise defined, the phrase "therapeutically effective amount" when used in connection with a 17α-hydroxylase/C₁₇₋₂₀-lyase inhibitor or therapeutic agent means an amount of the 17α-hydroxylase/C₁₇₋₂₀-lyase inhibitor or therapeutic agent effective for treating a disease or disorder disclosed herein, such as cancer.

As used herein and unless otherwise defined the phrase "refractory cancer," means cancer that is not responding to an anti-cancer treatment or cancer that is not responding sufficiently to an anti-cancer treatment. Refractory cancer can also include recurring or relapsing cancer.

As used herein and unless otherwise defined the phrase "refractory patient," means a patient who has refractory cancer.

As used herein and unless otherwise defined the phrase "relapse cancer," means cancer that was at one time responsive to an anti-cancer treatment but has become no longer responsive to such treatment or is no longer responding sufficiently to such treatment.

As used herein and unless otherwise defined the phrase "recurring cancer," means cancer that has returned after a patient has been earlier diagnosed with cancer, under gone treatment or had been previously diagnosed as cancer-free.

As used herein and unless otherwise defined the term "derivative" refers to a chemically modified compound wherein the chemical modification takes place at one or more functional groups of the compound. The derivative may retain or improve the pharmacological activity of the compound from which it is derived.

As used herein and unless otherwise defined the term "analog" refers to a chemical compound that is structurally similar to another but differs slightly in composition (as in the replacement of one atom by an atom of a different element or in the presence of a particular functional group).

As used herein and unless otherwise defined the phrase "pharmaceutically acceptable salt" refers to any salt of a 17α-hydroxylase/C_{17,20}-lyase inhibitor which retains the biological effectiveness of the 17α-hydroxylase/C_{17,20}-lyase inhibitor. Examples of pharmaceutically acceptable salts include, but are not limited to, acetates, sulfates, pyrosulfates, bisulfates, sulfites, bisulfites, phosphates, monohydrogenphosphates, dihydrogenphosphates, metaphosphates, pyrophosphates, chlorides, bromides, iodides, acetates, propionates, decanoates, caprylates, acrylates, formates, isobutyrates, caproates, heptanoates, propiolates, oxalates, malonates, succinates, suberates, sebacates, fumarates, maleates, butyne-1,4-dioates, hexyne-1,6-dioates, benzoates, chlorobenzoates, methylbenzoates, dinitrobenzoates, hydroxybenzoates, methoxybenzoates, phthalates, sulfonates, xylenesulfonates, phylacetates, phenylpropionates, phenylbutyrates, citrates, lactates, gamma-hydroxybutyrates, glycollates, tartarates, alkanesulfonates (e.g. methane-sulfonate or mesylate), propanesulfonates, naphthalene-1-sulfonates, naphthalene-2-sulfonates, and mandelates. Several of the officially approved salts are listed in Remington: The Science and Practice of Pharmacy, Mack Publ. Co., Easton.

### DETAILED DESCRIPTION OF THE INVENTION

The invention provides abiraterone acetate for use in a method of treating prostate cancer in a human, said method comprising administering to said human a therapeutically effective amount of abiraterone acetate and a therapeutically effective amount of prednisone.

The invention also provides prednisone for use in a method of treating prostate cancer in a human, said method comprising administering to said human a therapeutically effective amount of said prednisone and a therapeutically effective amount of abiraterone acetate.

The invention also provides a pharmaceutical composition for use in a method of treating prostate cancer in a human, wherein the pharmaceutical composition comprises a therapeutically effective amount of abiraterone acetate and a therapeutically effective amount of prednisone.

The methods described herein for treating cancer comprise administering to a mammal, preferably a human, a 17α-hydroxylase/C₁₇₋₂₀-lyase inhibitor in addition to at least one therapeutic agent, such as a steroid, particularly a glucocorticoid. The compositions described herein comprise a 17α-hydroxylase/C₁₇₋₂₀-lyase inhibitor and at least one additional therapeutic agent, such as a steroid, particularly a corticosteroid or glucocorticoid. Other anti-cancer treatments such as, administration of yet another anti-cancer agent, radiotherapy, chemotherapy, photodynamic therapy, surgery or other immunotherapy, can be used with the methods and compositions.

### 17α-hydroxylase/C₁₇₋₂₀-lyase Inhibitors

17α-hydroxylase/C₁₇₋₂₀-lyase inhibitors have been shown to be useful in the treatment of cancer, specifically hormone-dependent disorders such as, androgen-dependent and estrogen-dependent disorders like prostate cancer and breast cancer respectively, as described in United States Patent No. 5,604,213 to Barrie et al.*.*

The 17α-hydroxylase/C₁₇₋₂₀-lyase inhibitor comprises abiraterone acetate or 3β-acetoxy-17-(3-pyridyl)androsta-5,16-diene which has the following structural formula: 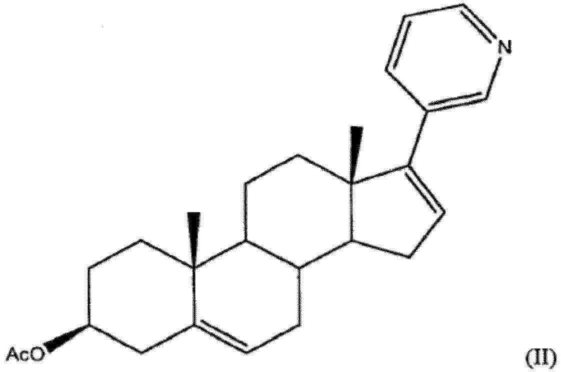 and pharmaceutically acceptable salts thereof.

Preferred salts of abiraterone acetate and methods of making such salts are also disclosed in United States Provisional Application No. 60/603,559 to Hunt. Preferred salts include, but are not limited to, acetates, citrates, lactates, alkanesulfonates (e.g. methane-sulfonate or mesylate) and tartarates. Of special interest is the abiraterone acetate mesylate salt (*i.e.* 3β-acetoxy-17-(3-pyridyl)androsta-5,16-diene mesylate salt) which has the following structural formula:

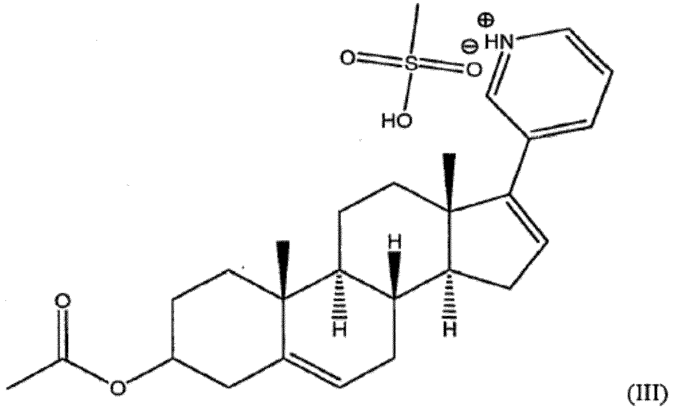

The 17α-hydroxylase/C₁₇₋₂₀-lyase inhibitors can be made according to any method known to one skilled in the art. For example, such inhibitors can be synthesized according to the method disclosed in United States Patent Nos. 5,604,213 and 5,618,807 to Barrie et al.*.* Another method of making 17α-hydroxylase/C₁₇₋₂₀-lyase inhibitors is disclosed in United States provisional application 60/603,558 to Bury.

### Additional Therapeutic Agents

The 17α-hydroxylase/C₁₇₋₂₀-lyase inhibitors are administered or combined with a steroid, such as a corticosteroid or a glucocorticoid. The 17α-hydroxylase/C₁₇₋₂₀-lyase inhibitors and the steroid may be administered in the same or in different compositions. A suitable steroid is prednisone. The amount of the steroid administered to a mammal having cancer is an amount that is sufficient to treat the cancer whether administered alone or in combination with a 17α-hydroxylase/C₁₇₋₂₀-lyase inhibitor.

In one embodiment, provided herein are compositions comprising both abiraterone acetate and prednisone. Steroids within the scope of the disclosure include, but are not limited to, (1) hydrocortisone (cortisol; cyprionate (*e.g*., CORTEF), oral; sodium phosphate injection (HYDROCORTONE PHOSPHATE); sodium succinate (*e.g*., A-HYDROCORT, Solu-CORTEF); cortisone acetate oral or injection forms, etc.), (2) dexamethasone (*e.g*., Decadron, oral; Decadron-LA injection, etc.), (3) prednisolone (*e.g*., Delta-CORTEF, prednisolone acetate (ECONOPRED), prednisolone sodium phosphate (HYDELTRASOL), prednisolone tebutate (HYDELTRA-TBA, etc.)), or (4) prednisone (*e.g.,* DELTASONE, etc.) and combinations thereof. See, *e.g.,* GOODMAN GILMAN's THE PHARMACOLOGICAL BASIS OF THERAPEUTICS, 10™ EDITION 2001.

In a specific aspect of the disclosure, single unit solid oral dosage forms which comprise an amount from about 50 mg to about 300 mg of abiraterone acetate and an amount from about 0.5 mg to about 3.0 mg of a steroid, *e.g*., glucocorticoid in a single composition, optionally with excipients, carriers, diluents, etc. is contemplated. For instance, the single unit dosage form can comprise about 250 mg of abiraterone acetate and about 1.0 mg, 1.25 mg, 1.5 mg, or 2.0 mg of a steroid.

### Administration of the 17α-hydroxylase/C_{17,20}-lyase Inhibitor and an Additional Therapeutic Agent

In certain aspects of the disclosure, the 17α-hydroxylase/C₁₇₋₂₀-lyase inhibitor and the additional therapeutic agent can be in separate compositions prior to administration.

In the alternative, the 17α-hydroxylase/C_{17,20}-lyase inhibitor and the additional therapeutic agent can be combined into a single composition for administration.

The 17α-hydroxylase/C_{17,20}-lyase inhibitor and the additional therapeutic agent can be administered sequentially or simultaneously. If administered sequentially, the order of administration is flexible. For instance, 17α-hydroxylase/C_{17,20}-lyase inhibitor acetate can be administered prior to administration of the additional therapeutic agent. Alternatively, administration of the additional therapeutic agent can precede administration of 17α-hydroxylase/C₁₇,₂₀-lyase inhibitor.

Whether they are administered as separate compositions or in one composition, each composition is preferably pharmaceutically suitable for administration. Moreover, the 17α-hydroxylase/C_{17,20}-lyase inhibitor and the therapeutic agent, if administered separately, can be administered by the same or different modes of administration. Examples of modes of administration include parenteral (*e.g*., subcutaneous, intramuscular, intraorbital, intracapsular, intraspinal, intrasternal, intravenous, intradermal, intraperitoneal, intraportal, intra-arterial, intrathecal, transmucosal, intra-articular, and intrapleural,), transdermal (*e.g*., topical), epidural, and mucosal (*e.g*., intranasal) injection or infusion, as well as oral, inhalation, pulmonary, and rectal administration. In specific embodiments, both are oral.

For example, the 17α-hydroxylase/C_{17,20}-lyase inhibitor can be administered transdermally and the additional therapeutic agent can be administered parenterally. Alternatively, the 17α-hydroxylase/C_{17,20}-lyase inhibitor can be administered orally, such as in a tablet, caplet or capsule, while the additional therapeutic agent can be administered intravenously. Such intravenous administered therapeutic agents include, but are not limited to, docetaxel injections, such as Taxotere®; paclitaxel injections, such as Paclitaxel® and mitoxantrone injections, such as Novantrone®. Also, the additional therapeutic agent can be in the form of depots or implants such as leuprolide depots and implants, *e*.*g*. Viadur® and Lupron Depot®; triptorelin depots, *e.g.* Trelstar®; goserelin implants, *e.g.* Zoladex®.

The suitable daily dosage of the 17α-hydroxylase/C_{17,20}-lyase inhibitor depends upon a number of factors, including, the nature of the severity of the condition to be treated, the particular inhibitor, the route of administration and the age, weight, and response of the individual patient. Suitable daily dosages of 17α-hydroxylase/C_{17,20}-lyase inhibitors can generally range from about 0.0001 mg/kg/day to about 1000 mg/kg/day, or from about 0.001 mg/kg/day to about 200 mg/kg/day, or from about 0.01 mg/kg/day to about 200 mg/kg/day, or from about 0.01 mg/kg/day to about 100 mg/kg/day in single or multiple doses.

In some aspects, the 17α-hydroxylase/C₁₇₋₂₀-lyase inhibitor can be administered in an amount from about 0.004 mg/day to about 5,000 mg/day, or from about 0.04 mg/day to about 3,000 mg/day, or from about 0.4 mg/day to about 1500 mg/day. In certain aspects, the 17α-hydroxylase/C₁₇₋₂₀-lyase inhibitor can be administered in an amount from about 0.1 mg/day to about 2000 mg/day or from about 1 mg/day to about 2000 mg/day or from about 50 mg/day to about 2000 mg/day or from about 100 mg/day to about 1500 mg/day or from about 5 mg/day to about 1,000 mg/day or from about 5 mg/day to about 900 mg/day or from about 10 mg/day to about 800 mg/day or from about 15 mg/day to about 700 mg/day or from about 20 mg/day to about 600 mg/day or from about 25 mg/day to about 500 mg/day in single or multiple doses.

The compositions for use in a treatment of a cancer in a mammal can comprise an amount of a 17α-hydroxylase/C₁₇₋₂₀-lyase inhibitor such as abiraterone acetate and an amount of a glucocorticoid such as, prednisone.

For example, the method can comprise administering an amount of about 50 mg/day to about 2000 mg/day of abiraterone acetate and an amount of about 0.01 mg/day to about 500 mg/day of prednisone. Also, the method can comprise administering an amount of about 500 mg/day to about 1500 mg/day of abiraterone acetate and an amount of about 10 mg/day to about 250 mg/day of prednisone.

### Compositions containing a 17α-hydroxylase/C₁₇₋₂₀-lyase inhibitor and an Additional Therapeutic Agent

In certain aspects of the disclosure, the compositions can contain a combination of 17α-hydroxylase/C₁₇₋₂₀-lyase inhibitor, preferably abiraterone acetate, and prednisone. Whether the 17α-hydroxylase/C₁₇₋₂₀-lyase inhibitor and the additional therapeutic agent are administered in separate compositions or as a single composition, the compositions can take various forms. For example, the compositions can take the form of solutions, suspensions, emulsions, tablets, pills, capsules, powders or sustained-release formulations, depending on the intended route of administration.

For topical or transdermal administration, the compositions can be formulated as solutions, gels, ointments, creams, suspensions or salves.

For oral administration, the compositions may be formulated as tablets, pills, dragees, troches, capsules, liquids, gels, syrups, slurries, suspensions and the like, for oral ingestion by a patient to be treated.

The composition may also be formulated in rectal or vaginal compositions such as suppositories or retention enemas that contain conventional suppository bases such as cocoa butter or other glycerides.

In addition to the formulations described previously, the composition may also be formulated as a depot preparation. Such long acting formulations may be administered by implantation (for example subcutaneously or intramuscularly) or by intramuscular injection. Thus, for example, the therapeutic agents may be formulated with suitable polymeric or hydrophobic materials (for example as an emulsion in an acceptable oil) or ion exchange resins, or as sparingly soluble derivatives, for example, as a sparingly soluble salt.

Additionally, the composition may be delivered using a sustained-release system, such as semi-permeable matrices of solid polymers containing the composition. Various forms of sustained-release materials have been established and are well known by those skilled in the art. Sustained-release capsules may, depending on their chemical nature can release the composition over a period of hours, days, weeks, months. For example a sustained release capsule can release the compositions over a period of 100 days or longer. Depending on the chemical nature and the biological stability of the composition, additional strategies for stabilization may be employed.

The compositions can further comprise a pharmaceutically acceptable carrier. The term "carrier" refers to a diluent, adjuvant (*e.g.*, Freund's adjuvant (complete and incomplete)), excipient, or vehicle with which the therapeutic is administered.

For parenteral administrations, the composition can comprise one or more of the following carriers: a sterile diluent such as water for injection, saline solution, fixed oils, polyethylene glycols, glycerin, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampules, disposable syringes or multiple dose vials made of glass or plastic.

For oral solid formulations suitable carriers include fillers such as sugars, e.g., lactose, sucrose, mannitol and sorbitol; cellulose preparations such as maize starch, wheat starch, rice starch, potato starch, gelatin, gum tragacanth, methyl cellulose, hydroxypropylmethyl-cellulose, sodium carboxymethylcellulose, fats and oils; granulating agents; and binding agents such as microcrystalline cellulose, gum tragacanth or gelatin; disintegrating agents, such as cross-linked polyvinylpyrrolidone, agar, or alginic acid or a salt thereof such as sodium alginate, Primogel, or corn starch; lubricants, such as magnesium stearate or Sterotes; glidants, such as colloidal silicon dioxide; a sweetening agent, such as sucrose or saccharin; or flavoring agents, such as peppermint, methyl salicylate, or orange flavoring. If desired, solid dosage forms may be sugar-coated or enteric-coated using standard techniques.

For intravenous administration, suitable carriers include physiological saline, bacteriostatic water, phosphate buffered saline (PBS). In all cases, the composition must be sterile and should be fluid to the extent that easy injectability with a syringe. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars; polyalcohols such as mannitol, sorbitol; sodium chloride in the composition. Prolonged absorption of the injectable compositions can be brought about by including in the composition an agent which delays absorption, for example, aluminum monostearate and gelatin.

Also for intravenous administration, the compositions may be formulated in solutions, preferably in physiologically compatible buffers such as Hanks's solution, Ringer's solution, or physiological saline buffer. The solution may contain formulatory agents such as suspending, stabilizing and/or dispersing agents. In a preferred embodiment, the compositions are formulated in sterile solutions.

For transmucosal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories.

For administration by inhalation, the compositions may be formulated as an aerosol spray from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. Capsules and cartridges of gelatin for use in an inhaler or insufflator may be formulated containing a powder mix of the composition and a suitable powder base such as lactose or starch.

The pharmaceutical compositions may be manufactured by means of conventional mixing, dissolving, granulating, dragee-making, levigating, emulsifying, encapsulating, entrapping or lyophilizing processes.

One example of a composition comprising a 17α-hydroxylase/C_{17,20}-lyase inhibitor and an additional therapeutic agent is an oral composition or composition suitable for oral administration comprising abiraterone acetate in combination with a steroid. For example, the oral composition can be a solid dosage form such as a pill, a tablet or a capsule. The oral composition can comprise about 10 mg, 25 mg, 50 mg, 75 mg, 100 mg, 150 mg, 200 mg, 250 mg, 300 mg, 350 mg, 400 mg, 450 mg, 500 mg, 550 mg, 600 mg, 650 mg, 700 mg, 750 mg, 800 mg, 850 mg, 900 mg, 950 mg, or 1000 mg of abiraterone acetate. The oral composition can comprises about 0.25 mg, 0.5 mg, 0.75 mg, 1.0 mg, 1.25 mg, 1.5 mg, 1.75 mg, 2.0 mg, 2.25 mg, 2.5 mg, 2.75 mg, 3.0 mg, 3.25 mg, 3.5 mg, 3.75 mg, 4.0 mg, 4.25 mg, 4.5 mg, 4.75 mg, 5.0 mg, 7.5 mg, 10 mg, 20 mg, 30 mg, 40 mg or 50 mg of a steroid, such as a glucocorticoid.

In one aspect, the oral composition can comprise about 50 mg to about 500 mg of abiraterone acetate and an amount of about 0.25 mg to about 3.5 mg of the steroid, such as prednisone. In other instances, the composition can comprise about 50 mg to about 300 mg of abiraterone acetate and an amount of about 1.0mg to about 2.5 mg of the steroid, such as prednisone. In another aspect of the disclosure. the composition can comprise about 50 mg to about 300 mg of abiraterone acetate and about 0.5 mg to about 3.0 mg of a steroid. For example, the oral composition can be a tablet containing 250 mg of abiraterone acetate; 1.25 mg or 2.0 mg of a steroid, such as prednisone; and one or more carriers, excipients, diluents or additional ingredients. Additionally, the oral composition can be a capsule containing 250 mg of abiraterone acetate; 1.25 mg or 2.0 mg of a steroid, prednisone; and one or more carriers, excipients, diluents or additional ingredients.

## Claims

1. Abiraterone acetate for use in a method of treating prostate cancer in a human, said method comprising administering to said human a therapeutically effective amount of abiraterone acetate and a therapeutically effective amount of prednisone.

2. Prednisone for use in a method of treating prostate cancer in a human, said method comprising administering to said human a therapeutically effective amount of said prednisone and a therapeutically effective amount of abiraterone acetate.

3. A pharmaceutical composition for use in a method of treating prostate cancer in a human, wherein the pharmaceutical composition comprises a therapeutically effective amount of abiraterone acetate and a therapeutically effective amount of prednisone.

## Patentansprüche

1. Abirateronacetat zur Verwendung bei einem Verfahren zur Behandlung von Prostatakrebs bei einem Menschen, bei dem man dem Menschen eine therapeutisch wirksame Menge von Abirateronacetat und eine therapeutisch wirksame Menge von Prednison verabreicht.

2. Prednison zur Verwendung bei einem Verfahren zur Behandlung von Prostatakrebs bei einem Menschen, bei dem man dem Menschen eine therapeutisch wirksame Menge von Prednison und eine therapeutisch wirksame Menge von Abirateronacetat verabreicht.

3. Pharmazeutische Zusammensetzung zur Verwendung bei einem Verfahren zur Behandlung von Prostatakrebs bei einem Menschen, wobei die pharmazeutische Zusammensetzung eine therapeutisch wirksame Menge von Abirateronacetat und eine therapeutisch wirksame Menge von Prednison umfasst.

## Revendications

1. Acétate d'abiratérone pour une utilisation dans un procédé de traitement d'un cancer de la prostate chez un humain, ledit procédé comprenant l'administration audit humain d'une quantité thérapeutiquement efficace d'acétate d'abiratérone et d'une quantité thérapeutiquement efficace de prednisone.

2. Prednisone pour une utilisation dans un procédé de traitement d'un cancer de la prostate chez un humain, ledit procédé comprenant l'administration audit humain d'une quantité thérapeutiquement efficace de ladite prednisone et d'une quantité thérapeutiquement efficace d'acétate d'abiratérone.

3. Composition pharmaceutique pour une utilisation dans un procédé de traitement d'un cancer de la prostate chez un humain, la composition pharmaceutique comprenant une quantité thérapeutiquement efficace d'acétate d'abiratérone et une quantité thérapeutiquement efficace de prednisone.
